(19) Europäisches Patentamt
European Patent Office
Office européen des brevets

(11) Veröffentlichungsnummer: **0 084 767**
**A2**

(12) **EUROPÄISCHE PATENTANMELDUNG**

(21) Anmeldenummer: **83100003.9**

(22) Anmeldetag: **03.01.83**

(51) Int. Cl.³: **C 07 D 251/50**, A 01 N 43/68

(30) Priorität: **23.01.82 DE 3202085**

(43) Veröffentlichungstag der Anmeldung: **03.08.83 Patentblatt 83/31**

(84) Benannte Vertragsstaaten: **AT BE CH DE FR GB IT LI LU NL SE**

(71) Anmelder: **CHEMISCHE WERKE HÜLS AG, Postfach 1320, D-4370 Marl 1 (DE)**

(72) Erfinder: **Geisler, Günter, Dr., Bachstrasse 1, D-4223 Voerde (DE)**
Erfinder: **Baltruschat, Helmut, Dr., Am Hagenbach 3, D-4405 Nottuln-Darup (DE)**
Erfinder: **Schnurbusch, Horst, Dr., Overwegstrasse 36, D-4690 Herne 1 (DE)**

(74) Vertreter: **Stell, Hanna, Dipl.-Chem., RSP PATENTE - PB 15 Postfach 1320, D-4370 Marl 1 (DE)**

(54) **Verwendung von Chlor-s-triazinen, die mit ein oder zwei Cyclohexylamino-Gruppen mit mindestens 3 einwertigen Substituenten oder mit Cyclopentylamino-Gruppen substituiert sind, als selektive Mittel gegen Unkräuter und Schadgräser.**

(57) Zur Vernichtung von Unkräutern und Schadgräsern in Nutzpflanzenkulturen wird vor und/oder nach dem Auflaufen der Saat das Ausbringen von Verbindungen aus der Gruppe der Chlor-cyclopentyl- bzw. cyclohexylamin-s-triazine der allgemeinen Formel

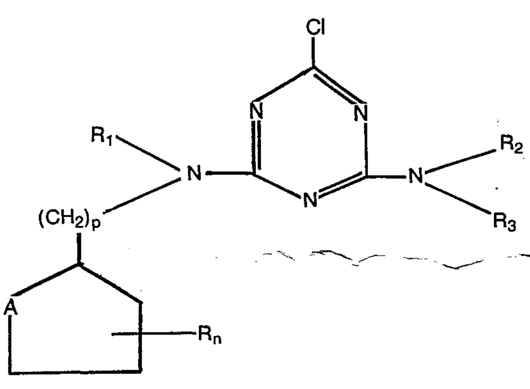

I,

in geeigneter Applikationsform in Mengen von 0,05 bis 10 kg/ha, vorzugsweise von 0,5 bis 2,5 kg/ha empfohlen.
Erklärung der Symbole im Text.

EP 0 084 767 A2

Verwendung von Chlor-s-triazinen, die mit ein oder zwei Cyclohexylamino-Gruppen mit mindestens 3 einwertigen Substituenten oder mit Cyclopentylamino-Gruppen substituiert sind, als selektive Mittel gegen Unkräuter und Schadgräser

S-Triazine wurden erstmals als Fungizide von der Ethyl Corp. New York, verwendet (DE-PS 965 608, US Prior. 31.08. 1951). Die empfohlenen Verbindungen enthalten ein (bis zwei) Chlor-Atome in 2- (und 4-) Stellung und - als Amin in 6- und 4- Stellung- gegebenenfalls substituierte - Aniline oder Naphthylamine.

Größeren Erfolg hatte das im Jahre 1954 von der Firma Geigy eingeführte Simazin ( = 2-Chlor-4,6 bis ethylamino-1,3,5-triazin) (CH-PS 329 277 und 342 784 bzw. DE-AS 10 11 904).

Simazin wirkt gegen sehr viele Unkräuter und Schadgräser, kann aber - insbesondere bei größeren Konzentrationen - auch Nutzpflanzen schädigen. (Auffällig ist lediglich die Selektivität gegenüber Mais).

Später kamen dann weitere s-Triazine auf den Markt. Als Standard-Produkte seien hier nur

Atrazin, das 2-Chlor-4-ethylamino-6-isopropylamino-1,3,5-triazin (DE-AS 10 11 904) und das Terbutryn, das 2 Methylthio-4-ethylamino-6 tert.butylamino-1,3,5-triazin (DE-AS 10 11 904, CH-PS 337 019, DE-AS 11 79 941 und 11 86 070 und 12 19 726) angeführt.

Die Cyclohexylgruppe als Aminsubstituent wurde 1962 erstmals von der NALCO Chemical Company in einem herbiziden

s-Triazin beansprucht (US-PS 31 94 646), allerdings ohne dafür auch Beispiele zu nennen. In entsprechender Weise wurde die Herstellung cyclohexylaminhaltiger Triazine auch von der Ciba-Geigy-AG 1965 unter Patentschutz gestellt (DE-AS 16 95 117).

Auf ihre herbizide Wirkung ausführlich untersucht wurden cyclohexylgruppenhaltige Chlor-s-triazine von der Nippon Kayaku K.K. (JA-OS 72-23436, Beispiele 1 bis 11).

Diese Firma hat später auch 2-Chlor-4-methylcyclohexylamino-s-triazine als algizide Verbindungen angemeldet und damit ein Verfahren zum Reinhalten von Rohrleitungen angeboten. (JA-OS 74-25135, nach Chem. Abstr. 81 (1974) 115,898)

Chlor-cycloalkylamino-triazine mit 3 bis 6 C-Atomen im Ring und der kennzeichnenden Gruppe-Z $(CH_2)_m-NR_2$ (mit Z = -O- oder -NH- , m = 2 oder 3 und R = $CH_3$ oder $C_2H_5$) wurden in der Textilindustrie von TEIJIN empfohlen (JA-OS 74 09 094, nach Chem.Abstr. 81 (1974) 92 907.)

Zur Steigerung der Wurzelausbeute und des Zuckergehaltes von Zuckerrüben schlägt Urad predsednictva Slovenskej akademie vied in Preßburg u.a. die Verwendung von Chlorcycloalkylamino-s-triazinen mit 3 bis 6 C-Atomen im Ring vor (DE-OS 28 33 582). Als Beispiel für eine Cycloalkylgruppe als Triazinsubstituent wird jedoch lediglich ein Cyclohexylamino-s-triazin mit einer Methylthiogruppe angeführt (l.c. Seite 26, Beispiel 44).

Ein Verfahren zur Herstellung derartiger Verbindungen gibt die Firma Ciba Geigy auch in US 41 86 265 an, ohne jedoch Beispiele für die $C_3$ - $C_6$-Cycloalkyl-

derivate anzuführen.

Der Nachteil der bisher bekannten Chlor-s-triazine war im allgemeinen ihre generelle Wirkung, d.h. auch die gegen Nutzpflanzen. Immerhin waren bei einzelnen s-Triazin-Derivaten ganz spezifische Selektivitäten aufgetreten (vgl. oben die von Simazin in Mais oder von Atrazin in Mais, Zuckerrohr oder Ananas oder von Terbutryn in Winterweizen, es fehlen jedoch solche Herbizide, die in Hafer, Gerste,Weizen, Reis, Raps usw. keine Schäden verursachen.

Eine überraschend hohe Selektivität gegenüber derartigen Nutzpflanzen konnte bei Chlor-s-triazinen beobachtet werden, die eine oder zwei, gegebenenfalls substituierte, Cyclopentylaminogruppen oder Cyclohexylaminogruppen mit mindestens drei einwertigen Substituenten in 4 (und 6)-Stellung des Triazinringes aufweisen.

Ähnliche Wirkungen zeigten die entsprechenden Derivate mit einer $CH_2$-Gruppe zwischen dem Ring und dem Aminstickstoff, sowie solche mit substituierten Cyclohexenaminen.

Gegenstand der Erfindung ist also die Verwendung von Chlor-cyclopentyl- bzw. cyclohexylamin-s-triazinen der allgemeinen Formel

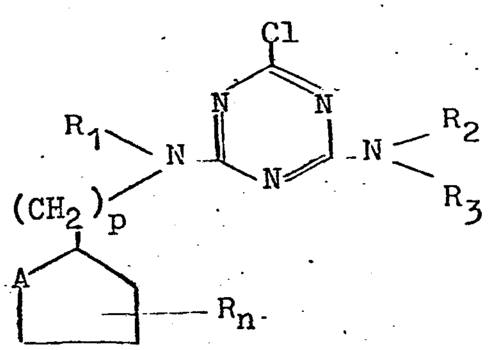

I,

in der

$R_1$ einen gerad- oder verzweigtkettigen Alkyl-, Alkoxy-Alkoxyalkyl- oder Alkylol-Rest oder - vorzugsweise - ein H-Atom,

$R_2$ einen Rest aus der Gruppe von $R_1$ oder einen

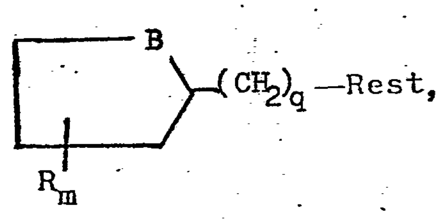

$R_3$ einen Rest aus der Gruppe von $R_1$, bevorzugt ein H-Atom, und

R einen Rest aus der Gruppe der Alkyle oder Alkoxide mit ein bis drei C-Atomen oder Hydroxyl oder - gewissermaßen als Derivat von 2 OH-Gruppen am selben C-Atom - die Oxogruppe bedeuten und

A = $-CH_2-$ , $-CH_2-CH_2-$ oder $-CH=CH-$,
B = $-CH_2-$ , $-CH_2-CH_2-$ oder $-CH=CH-$,

n für A = $-CH_2-$ jeweils 0 oder 1,2,3,4,5 oder 6 bzw. für A ≠ $-CH_2-$ jeweils 3,4,5 oder 6 und
m jeweils 0 oder 1,2,3,4,5 oder 6, sowie
p = entweder 0 oder 1 und
q = entweder 0 oder 1 sein können und
wobei von den Substituenten $R_1$ bis $R_3$ mindestens einer ein H-Atom sein muß, und wobei die n bzw. m Substituenten R an den gesättigten oder gegebenenfalls auch eine Doppelbindung aufweisenden Cycloalkylringen gleich oder verschieden sein können,

in geeigneter Applikationsform zur selektiven Behandlung von Nutzpflanzenkulturen mit breitem Wirkungsspektrum bei Unkräutern und Schadgräsern

vor und/oder nach dem Auflaufen der Saat.

Die Ketten, die von $R_1$,$R_2$ oder $R_3$ gebildet werden, sollten - bei Nichtberücksichtigung der H-Atomen und unabhängig vom Verzweigungsgrad - nicht mehr als 15 C-bzw. O-Atome aufweisen.

Bei der Zählung der Substituenten R rechnet eine Oxo-Gruppe doppelt, (eine $-CH_2-NH-$Gruppe jedoch gar nicht, denn diese wird durch die Kennzahl p bzw. q = 1 berücksichtigt).

Bei den Verbindungen gemäß der Formel I handelt es sich um Herbizide, welche sowohl im Vor- als auch im Nachauflaufverfahren (Tabellen 3 bis 5) ein breites Selektivitätsspektrum gegenüber zahlreichen Kulturpflanzen und eine sehr gute Wirkung gegen Schadgräser und zweikeimblättrige Unkräuter aufweisen.

Die vorliegenden Verbindungen sind im Vorauflaufverfahren

und/oder Nachauflaufverfahren selektiv in Soja (17*), Baumwolle (4), Reis (15), Mais (14) Winterraps (25), Gerste (8) und Weizen (22).

Die erfindungsgemäßen Verbindungen weisen ein breites Wirkungsspektrum gegen Schadgräser wie z.B. Ackerfuchsschwanz (1), Windhalm (24) und Flughafer (6) auf. Eine Reihe von zweikeimblättrigen Unkräutern wird ebenfalls erfaßt, z.B. Vogelmiere (20) Klettenlabkraut (13), Kamille (11, einschließlich Anthemis-Arten), Weißer Gänsefuß (21), Ackersenf (2), Taubnessel (18), Amaranth (3), Saatwucherblume (16), Klatschmohn (12), Knöterich (5), und Franzosenkraut (7).

Die Wirkstoffmenge kann von 0,0625 bis 10 kg/ha variieren, vorzugsweise von 0,5 bis 2,5 kg/ha. Im allgemeinen reichen jedoch auch schon 0,25 kg/ha für eine befriedigende Wirkung der reinen Stoffe aus. Die Mengenangabe verstehen sich selbstverständlich jeweils auf den reinen Wirkstoff je ha.

Trotz des breiten Wirkungsspektrums in den oben erwähnten Kulturen wurden sichtbare Schädigungen der Kulturpflanzen praktisch nicht beobachtet.

* Botanische Namen vgl. Tabelle 1, Seite 7.

(Anordnung in der Aufstellung nach dem Alphabet der deutschen Namen. Die Nummer hinter den deutschen Namen der vorliegenden Aufzählung auf dieser Seite erleichtert die Auffindung der betreffenden Pflanzennamen).

Tabelle 1

Botanische Namen der Testpflanzen:

| | deutsche Bezeichnung | | lateinische Bezeichnung |
|---|---|---|---|
| 1. | Ackerfuchsschwanz | = | Alopecurus myosuroides |
| 2. | Ackersenf | = | Sinapsis alba |
| 3. | Amaranth | = | Amaranthus retroflexus |
| 4. | Baumwolle | = | Gossypium hirsutum |
| 5. | Floh-Knöterich | = | Polygonum persicaria |
| 6. | Flughafer | = | Avena fatua |
| 7. | Franzosenkraut | = | Galinsoga parviflora |
| 8. | Gerste | = | Hordeum vulgare |
| 9. | Hafer | = | Avena sativa |
| 10. | Hühnerhirse | = | Echinocloa crus-galli |
| 11. | Kamille | = | Matricaria maritima |
| 12. | Klatschmohn | = | Papaver rhoeas |
| 13. | Klettenlabkraut | = | Galium aparine |
| 14. | Mais | = | Zea mays |
| 15. | Reis | = | Oryza sativa |
| 16. | Saatwucherblume | = | Chrysanthenum segetum |
| 17. | Soja | = | Glycine max |
| 18. | Taubnessel | = | Lamium purpureum |
| 19. | Tomate | = | Lycopersicum esculentum |
| 20. | Vogelmiere | = | Stellaria media |
| 21. | Weißer Gänsefuß | = | Chenopodium alba |
| 22. | Weizen | = | Triticum aestivum |
| 23. | Wicke | = | Vicia angustifolia |
| 24. | Windhalm | = | Apera spica-venti |
| 25. | Winterraps | = | Brassica napa |
| 26. | Zuckerrübe | = | Beta vulgaris |

Herstellung der erfindungsgemäßen Triazine

Die neuen Verbindungen der Formel I können nach an sich bekannten Methoden hergestellt werden, indem man Cyanurchlorid - vorzugsweise in einem Lösungsmittel oder Lösungsmittelgemisch, z.B. in Aceton, Aceton/Wasser, Dioxan, Dioxan/Wasser, Tetrahydrofuran oder in Dimethylformamid - mit den entsprechenden Aminen bei der jeweils zweckmäßigen Temperatur, die gewöhnlich zwischen -30 °C und +50 °C liegt, umsetzt.

Zur selektiven Substitution am Triazingerüst arbeitet man nach den für die stufenweise Substitution in 1,3,5-Triazinen bekannten Arbeitsweise.

Beispiel 1

2-Chlor-4-ethylamino-6-cyclopentylamino-s-triazin

In 250 Gew.-T. vorgelegtes Wasser (aqua dest.) wird eine bei Raumtemperatur hergestellte Lösung von 46 Gew.-T. Cyanurchlorid in 250 Gew.T. Aceton bei 0 °C zugetropft und die entstehende feine Suspension mit einer Kältemischung (vorzugsweise mit $CO_2$/Alkohol ) auf -10 °C abgekühlt.
In die so hergestellte Suspension werden 23 Gew.-T. Cyclopentylamin als 50 %-ige wäßrige Lösung unter starkem Rühren und Abführen der Reaktionswärme

eingetropft und eine halbe Stunde lang nachgerührt. Danach wird die äquivalente Menge einer 30 %-igen wässrigen Natronlauge tropfenweise in das saure Gemisch gegeben, bis sich ein pH-Wert von 8 bis 9 eingestellt hat.
Zur Zugabe der Natronlauge werden bei den obengenannten Einsatzmengen etwa 30 Min. benötigt. Das Reaktionsprodukt liegt als gutverteilte Suspension vor.

Darauf werden 12 Gew.-T. Ethylamin als 50 %-ige wässrige Lösung bei -10 °C unter kräftigem Rühren zugegeben. Anschließend wird die Temperatur auf 25 bis 30 °C erhöht und 1 Stunde bei dieser Temperatur nachgerührt.

Nach dieser Behandlung stellt sich ein pH-Wert von etwa 3 ein. Dann wird - im Laufe von etwa 2 Stunden - mit äquivalenten Mengen 30 %-iger wässriger Natronlauge neutralisiert. Gegen Ende der Neutralisation steigt der pH-Wert auf 9 an, während er sich vorher - bei etwa 80 % der eingesetzten Natronlauge-bei etwa 8 konstant hielt.

Darauf wird das im Reaktionsbrei vorhandene Aceton -zuletzt durch Strippen mit Stickstoff- bei 30 °C entfernt.
Das verbleibende weiße Produkt wird abgesaugt und der Niederschlag 4 bis 5 mal mit aqua dest. kochsalzfrei gewaschen. Darauf wird das körnige Produkt bei 50 °C im Vakuum getrocknet. Die Ausbeute beträgt etwa 95 % der Theorie.

In analoger Weise lassen sich andere Glieder der erfindungsgemäßen Cycloalkylamino -s-triazine herstellen. Eine Auswahl davon ist - einschließlich Beispiel 1 - in Tabelle 2 a,(S.12 u. 12 a) angeführt.

Darin bedeuten in den Beispielen 16 bis 20:
TMCP = 2,2,4- bzw. 2,4,4-Trimethylcyclopentyl-

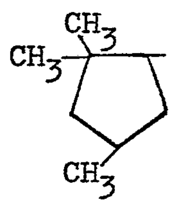

oder

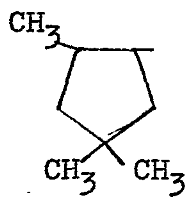

,

in den Beispielen 21 bis 26:
TMC = 3,3,5-Trimethylcyclohexyl

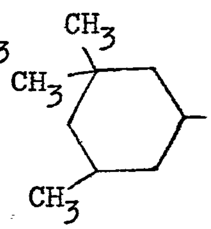

,

(Bei dieser Gruppe gibt es stabile cis- und trans-Formen und (cis-/trans-) Gemische.)

in Beispiel 27:
Δ 2,3-TMC EA = 1,5,5-Trimethyl-Δ 2,3-Cyclohexenyl-1 methylamino-

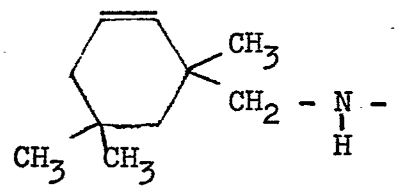

,

in Beispiel 28 bis 30:
IPAA = Isophoronaminoalkohol = = 1,5,5-Trimethyl-3-Oxy-cyclohexyl-1-methylamino-

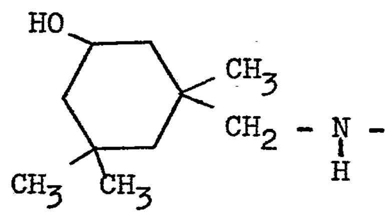

und in Beispiel 31 und 32:
IPEAA= Isophoronepoxidiaminoalkohol = 2,4,4-Trimethyl-2 Oxy-6-Oxo-cyclohexylamino-

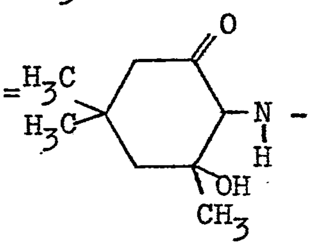

Die Tabelle 2 b ( S. 13 u. 13a) gibt die Substituenten dieser 33 Verbindungen in Bezug auf die Formel I an, sowie ihre Schmelzpunkte.

Tabelle 2a Liste der hergestellten Chlorcycloalkyl-s-triazine

Beispiel 1 = 2-Chlor-4-ethylamino-6-cyclopentylamino-s-triazin
Beispiel 2 = 2-Chlor-4-amino-6-cyclopentylamino-s-triazin
Beispiel 3 = 2-Chlor-4-methylamino-6-cyclopentylamino-s-triazin
Beispiel 4 = 2-Chlor-4-dimethylamino-6-cyclopentylamino-s-triazin
Beispiel 5 = 2-Chlor-4-isopropylamino-6-cyclopentylamino-s-triazin
Beispiel 6 = 2-Chlor-4-n-propylamino-6-cyclopentylamino-s-triazin
Beispiel 7 = 2-Chlor-4-ethanolamino-6-cyclopentylamino-s-triazin
Beispiel 8 = 2-Chlor-4-(2-methoxy-äthylamino)-6-cyclopentylamino-s-triazin
Beispiel 9 = 2-Chlor-4-(3-methoxypropylamino)-6-cyclopentylamino-s-triazin
Beispiel 10= 2-Chlor-4-(3-ethoxypropylamino)-6-cyclopentylamino-s-triazin
Beispiel 11= 2-Chlor-4-(3-n-butoxy-1-propylamino)-6-cyclopentylamino-s-triazin
Beispiel 12= 2-Chlor-4-(3-n-hexyloxy-1-propylamino)-6-cyclopentylamino-s-triazin
Beispiel 13= 2-Chlor-4-(3-isopropoxy-1-propylamino)-6-cyclopentylamino-s-triazin
Beispiel 14= 2-Chlor-4-(3-isobutoxy-1-propylamino)-6-cyclopentylamino-s-triazin
Beispiel 15= 2-Chlor-4-methoxy-6-cyclopentylamino-s-triazin
Beispiel 16= 2-Chlor-4-amino-6-TMCP-amino-s-triazin
Beispiel 17= 2-Chlor-4-methylamino-6-TMCP-amino-s-triazin
Beispiel 18= 2-Chlor-4-ethylamino-6-TMCP-amino-s-triazin
Beispiel 19= 2-Chlor-4-isopropylamino-6-TMCP-amino-s-triazin
Beispiel 20= 2-Chlor-4-(2-methoxy-ethylamino)-6-TMCP-amino-s-triazin
Beispiel 21= 2-Chlor-4-methylamino-6-trans-TMC -amino-s-triazin
Beispiel 22= 2-Chlor-4-isopropylamino-6-trans-TMC-amino-s-triazin
Beispiel 23= 2-Chlor-4-methylamino-6-cis-TMC-amino-s-triazin
Beispiel 24= 2-Chlor-4-dimethylamino-6-TMC-amino-s-triazin
Beispiel 25= 2-Chlor-4-ethylamino-6-TMC-amino-s-triazin
Beispiel 26= 2-Chlor-4-ethanolamino-6-cis-TMC-amino-s-triazin
Beispiel 27= 2-Chlor-4-isopropylamino-6- (Δ 2,3-TMC EA)-s-triazin

Beispiel 28= 2-Chlor-4-methylamino-6-IPAA-s-triazin
Beispiel 29= 2-Chlor-4-ethylamino-6-IPAA-s-triazin
Beispiel 30= 2-Chlor-4-isopropylamino-6-IPAA-s-triazin
Beispiel 31= 2-Chlor-4-ethylamino-6-IPEAA-s-triazin
Beispiel 32= 2-Chlor-4-isopropylamino-6-IPEAA-s-triazin
Beispiel 33= 2-Chlor-4,6-bis-cyclopentylamino-s-triazin

Tabelle 2 b

Liste der hergestellten Verbindungen mit ihren verschiedenen Substituenten und sonstigen Kenngrößen gemäß Formel I und ihren Schmelzpunkten

| Beispiel Nr. | $R_1$ | $R_2$ | $R_3$ | p | A | n | Stellung von $R_n$ | Schmelzbereich °C |
|---|---|---|---|---|---|---|---|---|
| 1 | H | H | $-CH_2$ $CH_3$ | 0 | $-CH_2-$ | 0 | -- | 185 - 188 |
| 2 | H | H | $-H$ | 0 | $-CH_2-$ | 0 | -- | 125 - 128 |
| 3 | H | H | $-CH_3$ | 0 | $-CH_2-$ | 0 | -- | 140 - 144 |
| 4 | H | $CH_3$ | $-CH_3$ | 0 | $-CH_2-$ | 0 | -- | 36 - 38 |
| 5 | H | H | $-CH=(CH_3)_2$ | 0 | $-CH_2-$ | 0 | -- | 162 - 164 |
| 6 | H | H | $-CH_2-CH_2-CH_3$ | 0 | $-CH_2-$ | 0 | -- | 163 - 167 |
| 7 | H | H | $-CH_2-CH_2-OH$ | 0 | $-CH_2-$ | 0 | -- | 115 - 117 |
| 8 | H | H | $-CH_2-CH_2-OCH_3$ | 0 | $-CH_2-$ | 0 | -- | 80 - 85 |
| 9 | H | H | $-CH_2-CH_2-CH_2OCH_3$ | 0 | $-CH_2$ | 0 | -- | 95 - 97 |
| 10 | H | H | $-CH_2-CH_2-CH_2OCH_2-CH_3$ | 0 | $-CH_2-$ | 0 | -- | 75 - 78 |
| 11 | H | H | $-CH_2-CH_2-CH_2OCH_2-CH_2-CH_2-CH_3$ | 0 | $-CH_2-$ | 0 | -- | 84 - 86 |
| 12 | H | H | $-CH_2-CH_2-CH_2-OCH_2CH_2-CH_2-CH_2-CH_2-CH_3$ | 0 | $-CH_2-$ | 0 | -- | 54 - 55 |
| 13 | H | H | $-CH_2-CH_2-CH_2-O-CH=(CH_3)_2$ | 0 | $-CH_2-$ | 0 | -- | 105 - 109 |
| 14 | H | H | $-CH_2-CH_2-CH_2-O-CH_2CH=(CH_3)_2$ | 0 | $-CH_2-$ | 0 | -- | 95 - 100 |

Fortsetzung von Tabelle 2 b

| Beispiel Nr. | $R_1$ | $R_2$ | $R_3$ | p | A | n | Stellung von $R_n$ R= $-CH_3$ / $-OH$ /=O | Schmelzbereich °C |
|---|---|---|---|---|---|---|---|---|
| 15 | H | H | $-OCH_3$ | 0 | $-CH_2-$ | 0 | -- | 53 - 55 |
| 16 | H | H | -H- | 0 | $-CH_2-$ | 3 | 2,2,4 (2,4,4) | 114 - 119 |
| 17 | H | H | $-CH_3$ | 0 | $-CH_2-$ | 3 | 2,2,4 (2,4,4) | 134 - 138 |
| 18 | H | H | $-CH_2-CH_3$ | 0 | $-CH_2-$ | 3 | 2,2,4 (2,4,4) | 94 - 98 |
| 19 | H | H | $-CH=(CH_3)_2$ | 0 | $-CH_2-$ | 3 | 2,2,4 (2,4,4) | 106 - 113 |
| 20 | H | H | $-CH_2-CH_2-O-CH_3$ | 0 | $-CH_2-$ | 3 | 2,2,4 (2,4,4) | 35- 40 |
| 21 | H | H | $-CH_3$ | 0 | $-CH_2-CH_2-$ | 3 | 3,3,5 (trans) | 139 - 144 |
| 22 | H | H | $-CH=(CH_3)_2$ | 0 | $-CH_2-CH_2-$ | 3 | 3,3,5 (trans) | 54 - 56 |
| 23 | H | H | $-CH_3$ | 0 | $-CH_2-CH_2-$ | 3 | 3,3,5 (cis) | 163 - 170 |
| 24 | H | $CH_3$ | $-CH_3$ | 0 | $-CH_2-CH_2-$ | 3 | 3,3,5 (Gemisch) | 107 - 115 |
| 25 | H | H | $-CH_2-CH_3-$ | 0 | $-CH_2-CH_2-$ | 3 | 3,3,5 " | 122 - 125 |
| 26 | H | H | $-CH_2-CH_2-OH$ | 0 | $-CH_2-CH_2-$ | 3 | 3,3,5 (cis) | 105 - 115 |
| 27 | H | H | $-CH=(CH_3)_2$ | 1 | $-CH=CH-$ | 3 | 1,5,5 | 122 - 127 |
| 28 | H | H | $-CH_3$ | 1 | $-CH_2-CH_2-$ | 4 | 1,5,5/3 | 169 - 173 |
| 29 | H | H | $-CH_2-CH_3$ | 1 | $-CH_2-CH_2-$ | 4 | 1,5,5/3 | 145 - 149 |
| 30 | H | H | $-CH=(CH_3)_2$ | 1 | $-CH_2-CH_2-$ | 4 | 1,5,5/3 | 105 - 109 |
| 31 | H | H | $-CH_2-CH_3-$ | 0 | $-CH_2-CH_2-$ | 6 | 2,4,4/2/6 | 156 - 160 |
| 32 | H | H | $-CH=(CH_3)_2$ | 0 | $-CH_2-CH_2-$ | 6 | 2,4,4/2/6 | 145 - 149 |
| 33 | H | H | 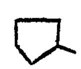 | 0 | $-CH_2-$ | 0 | -- | 193 - 195 |

Die erfindungsgemäßen Wirkstoffe können sowohl einzeln verwendet werden, als auch - bei verschiedenen Substituenten $R_1$, $R_2$, $R_3$ und/oder R, verschiedenen Bedeutungen von A und/oder B und/oder verschiedenen Werten von n,m und/oder p und/oder q in Mischung miteinander. Selbstverständlich kann man auch eine oder mehrere der genannten Wirkstoffe mit anderen Herbiziden, Fungiziden, Insektiziden, Wachstumsregulatoren usw. mischen und die jeweiligen Kulturpflanzen damit beaufschlagen.

Außerdem ist auch ein Zumischen zu Mineraldüngern möglich, am einfachsten zu Düngemittellösungen.

Üblicherweise werden den erfindungsgemäßen Wirkstoffen - gegebenenfalls in den oben erwähnten Mischungen - vor der Applikation Hilfsmittel aus der Gruppe der Träger-, Verdünnungs-Lösungs-,Netz-, Haft-, Dispergier- und/oder Emulgiermittel usw. zugesetzt und dadurch das Verspritzen, bzw. das Ermöglichen einer gleichmäßigen Verteilung der Wirkstoffe verbessert.

Applikation der Wirkstoffe

A Gewächshaus-Versuchsreihe mit 4 kg/ha

Für die (meisten der auf Seite 12 u. 12 a aufgeführten) erfindungsgemäßen Verbindungen wurden mit einer Aufwandmenge von 4 kg/ha, bezogen auf reinen Wirkstoff, entsprechende Gewächshausversuche für Ackersenf, Tomate, Flughafer, Hühnerhirse und Ackerfuchsschwanz durchgeführt.

Die jeweils benötigten Wirkstoffmengen wurden dabei in 1000 l Wasser/ha suspendiert.

Im Vorauflauf erfolgte die Applikation auf die Oberfläche vor dem Keimen der Samen und

im Nachauflauf auf die Blattoberfläche im zweiten bis dritten Blattstadium.

Drei Wochen nach der Behandlung ist bei den erfindungsgemäßen Verbindungen eine sehr gute Wirkung gegen zweikeimblättrige und einkeimblättrige Pflanzen sowohl im Vor- als auch im Nachauflauf (Tabellen 3a und 3 b) zu beobachten.

Die Auswertung der Versuchsergebnisse erfolgte durch die übliche Benotung, bei der
1 = volle Wirkung = Abtötung der Pflanzen und
5 = kein Wirkung = Pflanzen wie unbehandelt
bedeuten.

Überraschend ist bei einigen Verbindungen (Beispiele 4,5,15,26,27) die gute Verträglichkeit gegen Kreuzblütler z.B. Ackersenf, bei gleichzeitig sehr guter Wirkung gegen Gräser (Flughafer, oder Ackerfuchschwanz) im Vorauflauf.

Die Selektivität gegenüber Kreuzblütlern ist für einige Kulturen, z.B. für Winterraps äußerst interessant und für Wirkstoffe auf der Basis von s-Triazinen völlig überraschend.

B Gewächshausversuche mit geringeren Mengen und mit Vergleichsmitteln gemäß dem Stand der Technik

Für eine größere Anzahl von Testpflanzen wurden analoge Versuche wie bei der Reihe A mit Mengen von 2,1,1/2 und 1/4 kg Wirkstoff/ha durchgeführt. Auch dabei wurde die jeweilige Menge Wirkstoff in 1000 l Wasser/ha vor dem Aufbringen verteilt.

Wie die Tabellen 4 a + a' und 4 b + b'zeigen,ist sowohl im Vor- als auch im Nachauflauf für die erfindungsgemäßen Verbindungen eine hervorragende Wirkung gegen Schadgräser (z.B. Ackerfuchsschwanz, Flughafer, Windhalm und zweikeimblättrige Unkräuter zu beobachten.

Neben diesem breiten Wirkungsspektrum fällt aber die für Triazin-Verbindungen überraschend hohe Verträglichkeit in Getreidekulturen wie Gerste und/oder Weizen im Vorauflauf auf.

Die Verwendung einer erfindungsgemäßen Verbindung als Bodenherbizid in Getreidekulturen hat neben der spezifischen Wirkung gegen Schadgräser den Vorteil, daß - im Gegensatz zu den meisten bisher bekannten Bodenherbiziden - in Getreide gleichzeitig ein besonders weites Spektrum an zweikeimblättrigen Unkräutern erfaßt wird.

Ebenso überraschend für Triazin-Verbindung ist die Selektivität einiger erfindungsgemäßer Verbindungen

in Winterraps im Vorauflauf (vgl.Beispiel 2,5, 18, 19), gleichzeitig verbunden mit einer sehr guten Wirkung gegen Ackerfuchsschwanz und eine Reihe zweikeimblättriger Unkräuter.

Aus den Tabellen 4a bis 4b' geht darüber hinaus auch die gute Selektivität der erfindungsgemäßen Verbindungen in einigen wichtigen anderen Kulturen - wie Mais, Baumwolle, Soja, Reis - sowohl im Vor- als auch im Nachauflauf hervor.

C. Vergleich mit s-Triazinen vom Stand der Technik

Wegen der hohen herbiziden Wirkung der erfindungsgemäßen Verbindungen wurden die Aufwandmengen zusätzlich noch auf 1/8 und 1/16 kg/ha reduziert und im Gewächshausversuch gemeinsam mit Vergleichsmitteln aus der Gruppe der s-Triazine geprüft.

Als Standard-Vergleichsmittel wurde in diesen Versuchen Atrazin (= 2 Chlor-4-ethylamino-6-isopropylamino-s-triazin) und Terbutryn (= 2 Methylthio-4-ethylamin-2tert. butylamino-s-triazin) verwendet.

Die in dieser Versuchsserie ermittelte Tabelle 5 zeigt, daß die erfindungsgemäßen Verbindungen in wichtigen Indikationen (wie Ackerfuchsschwanz Windhalm, Vogelmiere oder Taubnessel in der herbiziden Wirkung die Verbindungen

des Standes der Technik übertreffen und darüber hinaus gleichzeitig eine hervorragende Kulturverträglichkeit, unter anderem in Getreide, gegenüber den genannten Vergleichsbeispielen besitzen.

Sowohl in dieser Versuchsreihe, wie auch bei der unter B, wurde bei Kulturhafer beobachtet, daß das Längenwachstum gehemmt wird, ohne daß Ertragsminderungen auftreten.

Vorteile der erfindungsgemäßen Verbindungen

Der Vorteil der erfindungsgemäßen Verbindungen liegt u.a. darin, daß die herbizide Wirkung des Vergleichsmittels (1)(Atrazin) z.B. gegen Ackerfuchsschwanz, Windhalm, Flughafer, Vogelmiere, Kamille und Taubnessel erreicht oder sogar übertroffen wird, daß aber andererseits - besonders im Vorauflauf-Verfahren - im Gegensatz zu diesem Vergleichsmittel eine hervorragende Selektivität in Getreide, vor allem in Gerste und Weizen, erzielt wird.

Das Vergleichsmittel 2 (Terbutryn), -ebenfalls aus der Gruppe der s-Triazine,-ist zwar für eine Vorauflaufanwendung in Wintergetreide in der Bundesrepublik zugelassen, jedoch

ist die Wirkung gegen in Getreide auftretende Schadgräser, wie Ackerfuchsschwanz und Windhalm, deutlich geringer als bei den erfindungsgemäßen Verbindungen (vgl. insbesondere Tabelle 4b).

Keines der Vergleichsmittel zeigt die bei einer Reihe der erfindungsgemäßen Verbindungen beobachtete Selektivität in Winterraps.

Die Längenwuchshemmung bei einigen Getreidearten erlaubt außerdem den Einsatz von einigen der erfindungsgemäßen Verbindungen als wachstumsregulierendes Mittel.

Darüber hinaus wurden bei einigen der neuen Verbindungen zusätzlich fungizide Wirkungen beobachtet.

Tabelle 3a: Herbizide Wirkung der erfindungsgemäßen Verbindungen auf verschiedene Testpflanzen im Vorauflauf

Aufwandmenge: 4 kg Wirkstoff/ha

| erfindungsgemäße Verbindungen | Ackersenf | Tomate | Flughafer | Hühnerhirse | Ackerfuchsschwanz |
|---|---|---|---|---|---|
| Beispiel 1 | 1 | 1 | 1 | 1 | 1 |
| " 2 | 1 | 1 | 1 | 2 | 1 |
| " 3 | 1 | 1 | 1 | 1 | 1 |
| " 4 | 5 | 1 | 2 | 3. | 1 |
| " 5 | 5 | 1 | 1 | 1 | 1 |
| " 6 | 5 | 1 | 3 | 5 | 4 |
| " 7 | 5 | 1 | 5 | 4 | 5 |
| " 8 | 1 | 1 | 1 | 1 | 1 |
| " 9 | 1 | 1 | 1 | 1 | 1 |
| " 10 | 2 | 1 | 1 | 2 | 1 |
| " 11 | 5 | 1 | 4 | 5 | 4 |
| " 12 | 5 | 2 | 5 | 5 | 4 |
| " 13 | 1 | 1 | 2 | 4 | 1 |
| " 14 | 5 | 1 | 4 | 5 | 4 |
| " 15 | 5 | 3 | 1 | 5 | 2 |
| " 16 | 3 | 1 | 1 | 1 | 1 |
| " 17 | 5 | 5 | 5 | 5 | 2 |
| " 18 | 3 | 1 | 1 | 1 | 1 |
| " 19 | 1 | 1 | 1 | 2 | 1 |
| " 20 | 1 | 1 | 1 | 3 | 1 |
| " 21 | 5 | 5 | 4 | 5 | 2 |
| " 22 | 4 | 1 | 4 | 4 | 2 |
| " 23 | 5 | 5 | 3 | 4 | 1 |
| " 24 | 5 | 5 | 4 | 4 | 2 |
| " 25 | 3 | 4 | 3 | 3 | 1 |
| " 26 | 5 | 1 | 2 | 3 | 1 |
| " 27 | 5 | 1 | 1 | 3 | 1 |
| " 28 | 2 | 1 | 1 | 3 | 2 |
| " 29 | 2 | 1 | 1 | 2 | 1 |
| " 30 | 4 | 3 | 4 | 5 | 1 |
| " 31 | 3 | 2 | 3 | 2 | 1 |
| " 32 | 5 | 4 | 1 | 3 | 1 |

Tabelle 3b: Herbizide Wirkung der erfindungsgemäßen Verbindungen auf verschiedene Testpflanzen im Nachauflauf

Aufwandmenge: 4 kg Wirkstoff/ha

| erfindungsgemäße Verbindungen | Ackersenf | Tomate | Flughafer | Hühnerhirse | Ackerfuchs-schwanz |
|---|---|---|---|---|---|
| Beispiel 1 | 1 | 1 | 1 | 1 | 1 |
| " 2 | 1 | 1 | 1 | 2 | 1 |
| " 3 | 1 | 1 | 1 | 1 | 1 |
| " 4 | 2 | 1 | 2 | 4 | 4 |
| " 5 | 1 | 1 | 2 | 3 | 1 |
| " 6 | 1 | 1 | 3 | 5 | 3 |
| " 7 | 1 | 1 | 1 | 4 | 1 |
| " 8 | 1 | 1 | 1 | 1 | 1 |
| " 9 | 1 | 1 | 1 | 1 | 1 |
| " 10 | 1 | 1 | 1 | 4 | 1 |
| " 11 | 1 | 1 | 5 | 4 | 4 |
| " 12 | 1 | 1 | 5 | 4 | 4 |
| " 13 | 1 | 1 | 4 | 5 | 1 |
| " 14 | 1 | 1 | 5 | 4 | 5 |
| " 15 | 3 | 2 | 1 | 3 | 3 |
| " 16 | 1 | 1 | 1 | 4 | 1 |
| " 17 | 1 | 3 | 4 | 5 | 4 |
| " 18 | 1 | 1 | 1 | 4 | 1 |
| " 19 | 2 | 1 | 1 | 4 | 2 |
| " 20 | 1 | 1 | 1 | 2 | 1 |
| " 21 | 1 | 4 | 5 | 5 | 2 |
| " 22 | 1 | 1 | 3 | 3 | 3 |
| " 23 | 1 | 1 | 1 | 4 | 1 |
| " 24 | 2 | 1 | 5 | 5 | 2 |
| " 25 | 1 | 1 | 5 | 5 | 3 |
| " 26 | 1 | 1 | 2 | 4 | 2 |
| " 27 | 1 | 3 | 5 | 5 | 4 |
| " 28 | 2 | 1 | 1 | 4 | 1 |
| " 29 | 1 | 1 | 4 | 3 | 3 |
| " 30 | 1 | 1 | 4 | 3 | 3 |
| " 31 | 3 | 1 | 1 | 3 | 3 |
| " 32 | 1 | 1 | 1 | 4 | 1 |

Tabelle 4a: Herbizide Wirkung der erfindungsgemäßen Verbindungen in den Aufwandmengen von 2, 1, 1/2 und 1/4 kg Wirkstoff/ha. Applikation vor dem Auflaufen der Pflanzen (Teil 1).

| Testpflanzen | Beispiel 1 kg Wirkstoff ha | | | | Beispiel 2 kg Wirkstoff ha | | | | Beispiel 3 kg Wirkstoff ha | | | | Beispiel 5 kg Wirkstoff ha | | | | Beispiel 8 kg Wirkstoff ha | | | | Beispiel 9 kg Wirkstoff ha | | | |
|---|---|---|---|---|---|---|---|---|---|---|---|---|---|---|---|---|---|---|---|---|---|---|---|---|
| | 2 | 1 | 1/2 | 1/4 | 2 | 1 | 1/2 | 1/4 | 2 | 1 | 1/2 | 1/4 | 2 | 1 | 1/2 | 1/4 | 2 | 1 | 1/2 | 1/4 | 2 | 1 | 1/2 | 1/4 |
| Ackersenf | 1 | 3 | 3 | 5 | 1 | 1 | 4 | 5 | 1 | 1 | 1 | 1 | 5 | 5 | 5 | 5 | 1 | 1 | 4 | 5 | 3 | 3 | 3 | 3 |
| Tomate | 1 | 1 | 1 | 2 | 1 | 1 | 4 | 4 | 1 | 1 | 1 | 1 | 1 | 1 | 1 | 4 | 1 | 1 | 1 | 1 | 1 | 1 | 1 | 1 |
| Hühnerhirse | 2 | 3 | 3 | 4 | 3 | 3 | 4 | 4 | 2 | 3 | 3 | 3 | 4 | 4 | 5 | 5 | 2 | 5 | 5 | 5 | 4 | 4 | 4 | 5 |
| Hafer | 1 | 1 | 2 | 3 | 1 | 1 | 1 | 1 | 1 | 1 | 2 | 3 | 2 | 2 | 5 | 5 | 1 | 2 | 5 | 5 | 1 | 1 | 4 | 4 |
| Flughafer | 1 | 1 | 1 | 1 | 1 | 1 | 1 | 1 | 1 | 1 | 1 | 1 | 1 | 3 | 3 | 5 | 1 | 1 | 2 | 2 | 1 | 1 | 2 | 5 |
| Ackerfuchsschwanz | 1 | 1 | 1 | 1 | 1 | 1 | 1 | 1 | 1 | 1 | 1 | 1 | 1 | 1 | 1 | 1 | 1 | 1 | 3 | 3 | 1 | 1 | 1 | 1 |
| Vogelmiere | 1 | 1 | 2 | 3 | 1 | 1 | 1 | 1 | 1 | 1 | 1 | 1 | 1 | 1 | 1 | 3 | 1 | 1 | 1 | 1 | 1 | 1 | 1 | 1 |
| Klettenlabkraut | 1 | 1 | 2 | 3 | 1 | 1 | 3 | 4 | 1 | 1 | 2 | 3 | 4 | 5 | 5 | 5 | 1 | 3 | 4 | 5 | 2 | 5 | 5 | 5 |
| Kamille | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Weißer Gänsefuß | 1 | 1 | 1 | 3 | 1 | 1 | 1 | 1 | 1 | 1 | 1 | 1 | 1 | 1 | 1 | 3 | 1 | 1 | 5 | 5 | 1 | 1 | 2 | 5 |
| Windhalm | 1 | 1 | 1 | 1 | 1 | 1 | 1 | 1 | 1 | 1 | 1 | 1 | 1 | 1 | 1 | 2 | 1 | 1 | 1 | 1 | 1 | 1 | 1 | 1 |
| Gerste | 3 | 5 | 5 | 5 | 3 | 4 | 5 | 5 | 3 | 4 | 4 | 5 | 4 | 5 | 5 | 5 | 1 | 1 | 4 | 5 | 1 | 1 | 3 | 4 |
| Weizen | 3 | 4 | 5 | 5 | 4 | 4 | 4 | 4 | 3 | 3 | 4 | 5 | 4 | 5 | 5 | 5 | 3 | 4 | 5 | 5 | 1 | 1 | 3 | 5 |

Tabelle 4a: Herbizide Wirkung der erfindungsgemäßen Verbindungen in den Aufwandmengen von 2, 1, 1/2 und 1/4 kg Wirkstoff/ha. Applikation vor dem Auflaufen der Pflanzen (Teil 2).

| Testpflanzen | Beispiel 1 kg Wirkstoff ha | | | | Beispiel 2 kg Wirkstoff ha | | | | Beispiel 3 kg Wirkstoff ha | | | | Beispiel 5 kg Wirkstoff ha | | | | Beispiel 8 kg Wirkstoff ha | | | | Beispiel 9 kg Wirkstoff ha | | | |
|---|---|---|---|---|---|---|---|---|---|---|---|---|---|---|---|---|---|---|---|---|---|---|---|---|
| | 2 | 1 | 1/2 | 1/4 | 2 | 1 | 1/2 | 1/4 | 2 | 1 | 1/2 | 1/4 | 2 | 1 | 1/2 | 1/4 | 2 | 1 | 1/2 | 1/4 | 2 | 1 | 1/2 | 1/4 |
| Taubnessel | 1 | 1 | 1 | 1 | 1 | 1 | 1 | 1 | 1 | 1 | 1 | 1 | 1 | 1 | 2 | 2 | 2 | 5 | 5 | 5 | 1 | 1 | 1 | 1 |
| Wicke | 5 | 5 | 5 | 5 | 1 | 4 | 4 | 4 | 3 | 3 | 5 | 5 | 5 | 5 | 5 | 5 | 5 | 5 | 5 | 5 | 5 | 5 | 5 | 5 |
| Amaranth | 1 | 1 | 1 | 1 | 1 | 1 | 1 | 1 | 1 | 1 | 1 | 1 | 1 | 1 | 1 | 1 | 1 | 1 | 3 | 3 | 1 | 1 | 3 | 3 |
| Zuckerrübe | 1 | 1 | 1 | 3 | 1 | 1 | 1 | 1 | 1 | 1 | 1 | 1 | 1 | 1 | 1 | 1 | 1 | 2 | 2 | 2 | 2 | 2 | 2 | 2 |
| Winterraps | 1 | 1 | 3 | 5 | 4 | 4 | 5 | 5 | 1 | 1 | 3 | 4 | 4 | 5 | 5 | 5 | 1 | 1 | 1 | 1 | 1 | 1 | 1 | 1 |
| Saatwucherblume | 1 | 1 | 1 | 1 | 1 | 1 | 1 | 1 | 1 | 1 | 1 | 1 | | | | | | | | | | | | |
| Klatschmohn | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Floh-Knöterich | 1 | 1 | 1 | 1 | 1 | 1 | 1 | 3 | 1 | 1 | 1 | 1 | 1 | 1 | 1 | 1 | 1 | 1 | 1 | 1 | 1 | 1 | 1 | 1 |
| Franzosenkraut | 1 | 1 | 1 | 1 | 1 | 1 | 1 | 3 | 1 | 1 | 1 | 1 | 1 | 1 | 1 | 1 | 1 | 1 | 1 | 1 | 1 | 1 | 1 | 1 |
| Soja | 3 | 4 | 5 | 5 | 3 | 4 | 4 | 4 | 3 | 4 | 5 | 5 | 3 | 3 | 4 | 5 | 3 | 4 | 5 | 5 | 4 | 4 | 5 | 5 |
| Baumwolle | 5 | 5 | 5 | 5 | 3 | 5 | 5 | 5 | 5 | 5 | 5 | 5 | 3 | 4 | 5 | 5 | 5 | 5 | 5 | 5 | 4 | 5 | 5 | 5 |
| Reis | 1 | 3 | 3 | 3 | 1 | 2 | 3 | 3 | 1 | 3 | 3 | 3 | 4 | 4 | 4 | 4 | 5 | 5 | 5 | 5 | 5 | 5 | 5 | 5 |
| Mais | 5 | 5 | 5 | 5 | 5 | 5 | 5 | 5 | 5 | 5 | 5 | 5 | 5 | 5 | 5 | 5 | 5 | 5 | 5 | 5 | 5 | 5 | 5 | 5 |

Tabelle 4a: Herbizide Wirkung der erfindungsgemäßen Verbindungen in den Aufwandmengen von 2, 1, 1/2 und 1/4 kg Wirkstoff/ha. Applikation vor dem Auflaufen der Pflanzen (Teil 1).

| Testpflanzen | Beispiel 18 kg Wirkstoff ha | | | | Beispiel 19 kg Wirkstoff ha | | | | Beispiel 29 kg Wirkstoff ha | | | |
|---|---|---|---|---|---|---|---|---|---|---|---|---|
| | 2 | 1 | 1/2 | 1/4 | 2 | 1 | 1/2 | 1/4 | 2 | 1 | 1/2 | 1/4 |
| Ackersenf | 3 | 3 | 4 | 4 | 5 | 5 | 5 | 5 | 2 | 3 | 3 | 3 |
| Tomate | 1 | 1 | 1 | 3 | 1 | 1 | 1 | 3 | 1 | 1 | 1 | 1 |
| Hühnerhirse | 2 | 3 | 5 | 5 | 4 | 4 | 5 | 5 | 2 | 4 | 5 | 5 |
| Hafer | 3 | 3 | 4 | 5 | 3 | 3 | 4 | 5 | 1 | 1 | 3 | 5 |
| Flughafer | 1 | 3 | 4 | 5 | 5 | 5 | 5 | 5 | 1 | 1 | 3 | 5 |
| Ackerfuchsschwanz | 1 | 1 | 1 | 3 | 1 | 1 | 2 | 3 | 1 | 1 | 2 | 3 |
| Vogelmiere | 1 | 1 | 1 | 1 | 1 | 1 | 1 | 1 | 1 | 1 | 1 | 1 |
| Klettenlabkraut | 3 | 3 | 4 | 4 | 4 | 4 | 4 | 5 | 5 | 5 | 5 | 5 |
| Kamille | 1 | 1 | 1 | 2 | 1 | 1 | 1 | 2 | 1 | 1 | 1 | 1 |
| Weißer Gänsefuß | 1 | 1 | 1 | 1 | 1 | 1 | 1 | 1 | 1 | 1 | 1 | 1 |
| Windhalm | 1 | 1 | 1 | 1 | 1 | 1 | 1 | 2 | 1 | 1 | 1 | 2 |
| Gerste | 5 | 5 | 5 | 5 | 5 | 5 | 5 | 5 | 5 | 5 | 5 | 5 |
| Weizen | 4 | 5 | 5 | 5 | 5 | 5 | 5 | 5 | 5 | 5 | 5 | 5 |

Tabelle 4a: Herbizide Wirkung der erfindungsgemäßen Verbindungen in den Aufwandmengen von 2, 1, 1/2 und 1/4 kg Wirkstoff/ha. Applikation vor dem Auflaufen der Pflanzen (Teil 2).

| Testpflanzen | Beispiel 18 kg Wirkstoff ha | | | | Beispiel 19 kg Wirkstoff ha | | | | Beispiel 29 kg Wirkstoff ha | | | |
|---|---|---|---|---|---|---|---|---|---|---|---|---|
| | 2 | 1 | 1/2 | 1/4 | 2 | 1 | 1/2 | 1/4 | 2 | 1 | 1/2 | 1/4 |
| Taubnessel | 1 | 1 | 1 | 3 | 1 | 1 | 2 | 4 | 2 | 2 | 3 | 3 |
| Wicke | 5 | 5 | 5 | 5 | 5 | 5 | 5 | 5 | 5 | 5 | 5 | 5 |
| Amaranth | 1 | 1 | 1 | 2 | 1 | 1 | 1 | 3 | 1 | 1 | 1 | 3 |
| Zuckerrübe | 1 | 1 | 4 | 4 | 1 | 2 | 4 | 4 | 5 | 5 | 5 | 5 |
| Winterraps | 5 | 5 | 5 | 5 | 5 | 5 | 5 | 5 | 1 | 2 | 4 | 5 |
| Saatwucherblume | 1 | 1 | 1 | 1 | 1 | 1 | 1 | 1 | 1 | 1 | 1 | 1 |
| Klatschmohn | 1 | 1 | 1 | 1 | 1 | 1 | 1 | 1 | 1 | 1 | 1 | 1 |
| Floh-Knöterich | 2 | 2 | 3 | 3 | 2 | 2 | 3 | 4 | 1 | 2 | 3 | 4 |
| Franzosenkraut | 1 | 1 | 1 | 1 | 1 | 1 | 1 | 1 | 1 | 1 | 1 | 1 |
| Soja | 3 | 4 | 5 | 5 | 4 | 5 | 5 | 5 | 4 | 5 | 5 | 5 |
| Baumwolle | 5 | 5 | 5 | 5 | 5 | 5 | 5 | 5 | 5 | 5 | 5 | 5 |
| Reis | 5 | 5 | 5 | 5 | 5 | 5 | 5 | 5 | 5 | 5 | 5 | 5 |
| Mais | 5 | 5 | 5 | 5 | 5 | 5 | 5 | 5 | 5 | 5 | 5 | 5 |

Tabelle 4b: Herbizide Wirkung der erfindungsgemäßen Verbindungen in den Aufwandmengen von 2, 1, 1/2 und 1/4 kg Wirkstoff/ha. Applikation nach dem Auflaufen der Pflanzen (Teil 1).

| Testpflanzen | Beispiel 1 kg Wirkstoff ha | | | | Beispiel 2 kg Wirkstoff ha | | | | Beispiel 3 kg Wirkstoff ha | | | | Beispiel 5 kg Wirkstoff ha | | | | Beispiel 8 kg Wirkstoff ha | | | | Beispiel 9 kg Wirkstoff ha | | | |
|---|---|---|---|---|---|---|---|---|---|---|---|---|---|---|---|---|---|---|---|---|---|---|---|---|
| | 2 | 1 | 1/2 | 1/4 | 2 | 1 | 1/2 | 1/4 | 2 | 1 | 1/2 | 1/4 | 2 | 1 | 1/2 | 1/4 | 2 | 1 | 1/2 | 1/4 | 2 | 1 | 1/2 | 1/4 |
| Ackersenf | 1 | 1 | 1 | 1 | 1 | 3 | 4 | 4 | 1 | 1 | 1 | 1 | 1 | 1 | 2 | 4 | 1 | 1 | 1 | 1 | 1 | 1 | 1 | 1 |
| Tomate | 1 | 1 | 1 | 1 | 1 | 1 | 3 | 4 | 1 | 1 | 1 | 1 | 1 | 1 | 1 | 5 | 1 | 1 | 1 | 1 | 1 | 1 | 1 | 1 |
| Hühnerhirse | 1 | 2 | 3 | 3 | 5 | 5 | 5 | 5 | 1 | 2 | 2 | 3 | 4 | 4 | 4 | 4 | 2 | 5 | 5 | 5 | 3 | 3 | 4 | 4 |
| Hafer | 1 | 1 | 3 | 4 | 1 | 1 | 1 | 2 | 1 | 1 | 3 | 4 | 1 | 1 | 1 | 5 | 1 | 1 | 4 | 5 | 1 | 1 | 3 | 4 |
| Flughafer | 1 | 1 | 1 | 1 | 1 | 1 | 2 | 2 | 1 | 1 | 1 | 1 | 1 | 1 | 2 | 4 | 1 | 1 | 2 | 4 | 1 | 1 | 1 | 1 |
| Ackerfuchsschwanz | 1 | 1 | 1 | 1 | 1 | 1 | 1 | 1 | 1 | 1 | 1 | 1 | 1 | 1 | 1 | 1 | 1 | 1 | 2 | 4 | 1 | 1 | 1 | 1 |
| Vogelmiere | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Klettenlabkraut | 1 | 1 | 2 | 3 | 1 | 1 | 4 | 4 | 1 | 1 | 2 | 2 | 1 | 1 | 2 | 3 | 1 | 2 | 4 | 4 | 1 | 1 | 4 | 5 |
| Kamille | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Weißer Gänsefuß | 1 | 1 | 1 | 1 | 1 | 1 | 2 | 4 | 1 | 1 | 1 | 1 | 1 | 1 | 1 | 1 | 1 | 1 | 1 | 1 | 1 | 1 | 1 | 1 |
| Windhalm | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Gerste | 1 | 1 | 2 | 3 | 1 | 1 | 4 | 4 | 1 | 1 | 2 | 3 | 4 | 5 | 5 | 5 | 1 | 3 | 3 | 4 | 1 | 1 | 1 | 1 |
| Weizen | 1 | 4 | 2 | 4 | 3 | 4 | 4 | 4 | 1 | 1 | 3 | 3 | 3 | 3 | 4 | 4 | 2 | 4 | 5 | 5 | 1 | 1 | 1 | 1 |

O.Z. 37..-H

Tabelle 4 b: Herbizide Wirkung der erfindungsgemäßen Verbindungen in den Aufwandmengen von 2, 1, 1/2 und 1/4 kg Wirkstoff/ha. Applikation nach dem Auflaufen der Pflanzen (Teil 2).

| Testpflanzen | Beispiel 1 kg Wirkstoff ha | | | | Beispiel 2 kg Wirkstoff ha | | | | Beispiel 3 kg Wirkstoff ha | | | | Beispiel 5 kg Wirkstoff ha | | | | Beispiel 8 kg Wirkstoff ha | | | | Beispiel 9 kg Wirkstoff ha | | | |
|---|---|---|---|---|---|---|---|---|---|---|---|---|---|---|---|---|---|---|---|---|---|---|---|---|
| | 2 | 1 | 1/2 | 1/4 | 2 | 1 | 1/2 | 1/4 | 2 | 1 | 1/2 | 1/4 | 2 | 1 | 1/2 | 1/4 | 2 | 1 | 1/2 | 1/4 | 2 | 1 | 1/2 | 1/4 |
| Taubnessel | 1 | 1 | 1 | 1 | 1 | 1 | 1 | 1 | 1 | 1 | 1 | 1 | 1 | 1 | 3 | 4 | 1 | 1 | 1 | 1 | 1 | 1 | 1 | 1 |
| Wicke | 3 | 4 | 5 | 5 | 4 | 4 | 4 | 4 | 3 | 3 | 5 | 5 | 4 | 4 | 5 | 5 | 5 | 5 | 5 | 5 | 5 | 5 | 5 | 5 |
| Amaranth | 1 | 1 | 1 | 2 | 1 | 1 | 1 | 2 | 1 | 1 | 1 | 1 | 2 | 2 | 3 | 4 | 2 | 2 | 3 | 3 | 1 | 1 | 1 | 1 |
| Zuckerrübe | 1 | 1 | 1 | 1 | 1 | 1 | 1 | 1 | 1 | 1 | 1 | 1 | 1 | 1 | 3 | 5 | 1 | 2 | 2 | 2 | 1 | 1 | 1 | 1 |
| Winterraps | 1 | 1 | 1 | 1 | 1 | 1 | 1 | 3 | 1 | 1 | 1 | 1 | 1 | 1 | 1 | 3 | 1 | 1 | 4 | 4 | 1 | 1 | 1 | 1 |
| Saatwucherblume | 1 | 1 | 1 | 1 | 1 | 1 | 1 | 1 | 1 | 1 | 1 | 1 | | | | | | | | | | | | |
| Klatschmohn | 1 | 1 | 1 | 1 | 1 | 1 | 1 | 1 | 1 | 1 | 1 | 1 | 1 | 1 | 1 | 3 | 1 | 1 | 1 | 1 | 1 | 1 | 1 | 1 |
| Floh-Knöterich | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Franzosenkraut | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Soja | 3 | 3 | 4 | 4 | 3 | 3 | 5 | 5 | 3 | 3 | 4 | 5 | 3 | 4 | 4 | 5 | 3 | 5 | 5 | 5 | 3 | 5 | 5 | 5 |
| Baumwolle | 3 | 4 | 4 | 4 | 3 | 4 | 4 | 5 | 3 | 4 | 4 | 4 | 3 | 5 | 5 | 5 | 3 | 4 | 5 | 5 | 5 | 5 | 5 | 5 |
| Reis | 4 | 4 | 5 | 5 | 4 | 4 | 5 | 5 | 4 | 4 | 5 | 5 | 3 | 4 | 5 | 5 | 5 | 5 | 5 | 5 | 5 | 5 | 5 | 5 |
| Mais | 5 | 5 | 5 | 5 | 5 | 5 | 5 | 5 | 5 | 5 | 5 | 5 | 5 | 5 | 5 | 5 | 5 | 5 | 5 | 5 | 5 | 5 | 5 | 5 |

Tabelle 4b: Herbizide Wirkung der erfindungsgemäßen Verbindungen in den Aufwandmengen von 2, 1, 1/2 und 1/4 kg Wirkstoff/ha. Applikation nach dem Auflaufen der Pflanzen (Teil 1).

| Testpflanzen | Beispiel 18 kg Wirkstoff ha | | | | Beispiel 19 kg Wirkstoff ha | | | | Beispiel 29 kg Wirkstoff ha | | | |
|---|---|---|---|---|---|---|---|---|---|---|---|---|
| | 2 | 1 | 1/2 | 1/4 | 2 | 1 | 1/2 | 1/4 | 2 | 1 | 1/2 | 1/4 |
| Ackersenf | 1 | 1 | 1 | 1 | 1 | 2 | 3 | 4 | 1 | 1 | 1 | 1 |
| Tomate | 1 | 1 | 1 | 1 | 1 | 1 | 2 | 2 | 1 | 1 | 1 | 1 |
| Hühnerhirse | 1 | 1 | 3 | 5 | 1 | 1 | 2 | 3 | 2 | 4 | 5 | 5 |
| Hafer | 2 | 3 | 4 | 5 | 5 | 5 | 5 | 5 | 1 | 1 | 1 | 3 |
| Flughafer | 1 | 1 | 2 | 3 | 1 | 3 | 3 | 5 | 1 | 1 | 1 | 3 |
| Ackerfuchsschwanz | 1 | 1 | 1 | 1 | 1 | 1 | 2 | 3 | 1 | 1 | 2 | 3 |
| Vogelmiere | 1 | 1 | 1 | 1 | 1 | 1 | 1 | 1 | 1 | 1 | 1 | 1 |
| Klettenlabkraut | 2 | 2 | 3 | 4 | 4 | 5 | 5 | 5 | 5 | 5 | 5 | 5 |
| Kamille | 1 | 1 | 1 | 1 | 1 | 1 | 1 | 1 | 1 | 1 | 1 | 1 |
| Weißer Gänsefuß | 1 | 1 | 1 | 1 | 1 | 1 | 1 | 1 | 1 | 1 | 2 | 4 |
| Windhalm | 1 | 1 | 1 | 1 | 1 | 1 | 3 | 3 | 1 | 1 | 1 | 3 |
| Gerste | 4 | 5 | 5 | 5 | 5 | 5 | 5 | 5 | 4 | 5 | 5 | 5 |
| Weizen | 3 | 5 | 5 | 5 | 5 | 5 | 5 | 5 | 5 | 5 | 5 | 5 |

**Tabelle 4b:** Herbizide Wirkung der erfindungsgemäßen Verbindungen in den Aufwandmengen von 2, 1, 1/2 und 1/4 kg Wirkstoff/ha. Applikation nach dem Auflaufen der Pflanzen (Teil 2).

| Testpflanzen | Beispiel 18 kg Wirkstoff ha | | | | Beispiel 19 kg Wirkstoff ha | | | | Beispiel 29 kg Wirkstoff ha | | | |
|---|---|---|---|---|---|---|---|---|---|---|---|---|
| | 2 | 1 | 1/2 | 1/4 | 2 | 1 | 1/2 | 1/4 | 2 | 1 | 1/2 | 1/4 |
| Taubnessel | 1 | 1 | 1 | 1 | 1 | 2 | 3 | 3 | 1 | 1 | 3 | 3 |
| Wicke | 4 | 5 | 5 | 5 | 5 | 5 | 5 | 5 | 2 | 4 | 4 | 5 |
| Amaranth | 1 | 1 | 2 | 3 | 1 | 1 | 2 | 3 | 1 | 1 | 3 | 3 |
| Zuckerrübe | 1 | 1 | 1 | 1 | 1 | 2 | 3 | 5 | 2 | 2 | 2 | 2 |
| Winterraps | 1 | 1 | 1 | 1 | 5 | 5 | 5 | 5 | 1 | 1 | 1 | 3 |
| Klatschmohn | 1 | 1 | 1 | 1 | 1 | 1 | 1 | 1 | 1 | 1 | 1 | 1 |
| Floh-Knöterich | 1 | 1 | 1 | 1 | 1 | 1 | 1 | 1 | 1 | 1 | 1 | 3 |
| Franzosenkraut | 1 | 1 | 1 | 1 | 1 | 1 | 1 | 1 | 1 | 1 | 1 | 1 |
| Soja | 3 | 3 | 5 | 5 | 3 | 3 | 5 | 5 | 3 | 3 | 4 | 4 |
| Baumwolle | 3 | 4 | 4 | 5 | 3 | 4 | 4 | 5 | 5 | 5 | 5 | 5 |
| Reis | 2 | 4 | 4 | 4 | 2 | 4 | 4 | 5 | 2 | 4 | 4 | 5 |
| Mais | 5 | 5 | 5 | 5 | 5 | 5 | 5 | 5 | 5 | 5 | 5 | 5 |

<u>Tabelle 5:</u> Herbizide Wirkung der erfindungsgemäßen Verbindungen in den Aufwandmengen von 0,125 und 0,0625 kg/ha nach Applikation <u>vor dem Auflaufen</u> (VA) bzw. <u>nach dem Auflaufen</u> (NA) der Testpflanzen

| Verbindung | kg Wirkstoff/ha | Ackerfuchsschwanz | | Windhalm | | Flughafer | | Vogelmiere | | Kamille | | Hafer | | Gerste | | Weizen | | Wi-Raps | |
|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA |
| Beispiel 1 | 0,125 | 1 | 1 | 1 | 3 | 1 | 1 | 1 | 1 | 1 | 1 | 3 | 4 | 4 | 3 | 5 | 5 | 5 | 5 |
| | 0,0625 | 2 | 2 | 2 | 4 | 1 | 4 | 1 | 1 | 1 | 1 | 5 | 5 | 3 | 4 | 5 | 5 | 5 | 5 |
| Beispiel 2 | 0,125 | 1 | 1 | 1 | 1 | 1 | 2 | 2 | 2 | 1 | 1 | 2 | 2 | 4 | 5 | 5 | 4 | 5 | 4 |
| | 0,0625 | 1 | 2 | 1 | 1 | 1 | 3 | 2 | 2 | 1 | 1 | 2 | 2 | 4 | 5 | 5 | 4 | 5 | 4 |
| Beispiel 3 | 0,125 | 1 | 1 | 1 | 2 | 1 | 1 | 1 | 1 | 1 | 1 | 3 | 4 | 4 | 3 | 5 | 5 | 5 | 3 |
| | 0,0625 | 2 | 1 | 1 | 3 | 2 | 3 | 1 | 1 | 1 | 1 | 5 | 4 | 5 | 3 | 5 | 5 | 5 | 3 |
| Vergleichsmittel 1 | 0,125 | 2 | 1 | 1 | 3 | 4 | 1 | 1 | 2 | 1 | 1 | 2 | 1 | 3 | 2 | 2 | 2 | 3 | 1 |
| | 0,0625 | 3 | 3 | 2 | 4 | 5 | 2 | 3 | 3 | 2 | 1 | 3 | 1 | 3 | 3 | 3 | 2 | 4 | 2 |
| Vergleichsmittel 2 | 0,125 | 2 | 1 | 4 | 3 | 4 | 2 | 3 | 1 | 1 | 1 | 4 | 2 | 3 | 2 | 4 | 1 | 5 | 1 |
| | 0,0625 | 3 | 3 | 4 | 4 | 5 | 4 | 5 | 1 | 2 | 1 | 5 | 5 | 5 | 4 | 5 | 4 | 5 | 2 |

Vergleichsmittel 1 = Atrazin
" 2 = Terbutryn

<u>Patentansprüche</u> O.Z. 3790-H

1. Verwendung von Chlor-cyclopentyl- bzw. cyclohexylamin-s-triazinen der allgemeinen Formel

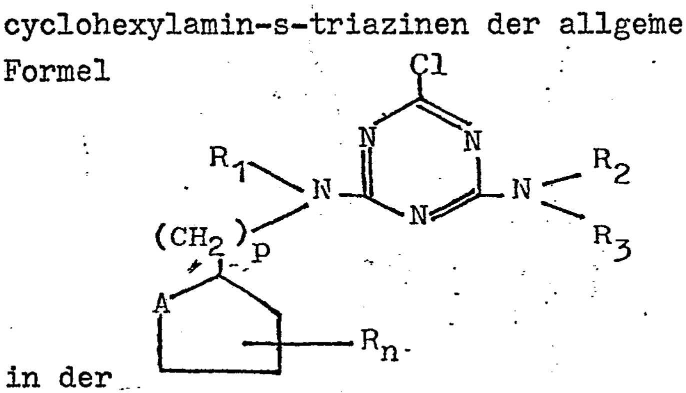

I ,

in der

$R_1$ einen gerad- oder verzweigtkettigen Alkyl-, Alkoxy-, Alkoxyalkyl oder Alkylol-Rest oder - vorzugsweise - ein H-Atom,

$R_2$ einen Rest aus der Gruppe von $R_1$ oder einen

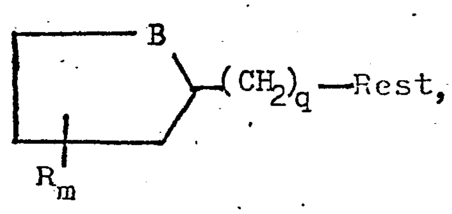

$R_3$ einen Rest aus der Gruppe von $R_1$, bevorzugt ein H-Atom und

R einen Rest aus der Gruppe der Alkyle oder Alkoxide mit ein bis drei C-Atomen oder Hydroxyl oder - gewissermaßen als Derivat von 2 OH-Gruppen am selben C-Atom - die Oxogruppe bedeuten und

A = $-CH_2-$, $-CH_2-CH_2-$ oder $-CH=CH-$,
B = $-CH_2-$, $-CH_2-CH_2-$ oder $-CH=CH-$,

n für A = $-CH_2-$ jeweils 0 oder 1,2,3,4,5 oder 6 bzw. für A $\neq$ $-CH_2-$ jeweils 3,4,5 oder 6 und
m jeweils 0 oder 1,2,3,4,5 oder 6, sowie
p = entweder 0 oder 1 und
q = entweder 0 oder 1 sein können und

wobei von den Substituenten $R_1$ bis $R_3$ mindestens einer ein H-Atom sein muß, und wobei die n bzw. m Substituenten R an den gesättigten oder gegebenenfalls auch eine Doppelbindung aufweisenden Cycloalkylringen gleich oder verschieden sein können,

in geeigneter Applikationsform zur selektiven Behandlung von Nutzpflanzenkulturen mit breitem Wirkungsspektrum bei Unkräutern und Schadgräsern

vor und/oder nach dem Auflaufen der Saat.

2. Verwendung von Mischungen von zwei oder mehreren Wirkstoffen nach Anspruch 1 gemäß Formel I, bei denen sich die Einzelwirkstoffe durch unterschiedlichen Substituenten $R_1$, $R_2$, $R_3$ und/oder R, bzw. A und/oder B und/oder die Werte von n und/oder m und/oder p und/oder q voneinander unterscheiden.

3. Verwendung eines oder mehrerer der Wirkstoffe nach Anspruch 1 oder 2 in Mengen von je 0,05 bis 10 kg/ha, vorzugsweise von 0,5 bis 2,5 kg/ha.

4. Verwendung eines oder mehrerer der Wirkstoffe nach Anspruch 1 oder 2 und 3,

dadurch gekennzeichnet, daß man die jeweilige Applikationsform durch Zumischen von Hilfsmitteln aus der Gruppe der Träger-, Verdünnungs-, Lösungs-, Netz-, Haft-, Dispergier- und Emulgiermittel gewinnt.

5. Verwendung eines oder mehrerer der Wirkstoffe nach Anspruch 1 und einem oder mehreren der Ansprüche 2 bis 4, gekennzeichnet durch die Zumischung von Stoffen aus der Gruppe der Herbizide, Fungizide, Insektizide, Wachstumsregulatoren usw..

6. Verwendung eines oder mehrerer der Wirkstoffe nach Anspruch 1 und gegebenenfalls nach einem oder mehreren der Ansprüche 2 bis 5 als Zusatz zu Mineraldüngern.

7473/40-ri